# EUROPEAN PATENT SPECIFICATION

(11) **EP 3 165 221 B1**
(45) Date of publication and mention of the grant of the patent: **30.12.2020**
(21) Application number: 15815344.5
(22) Date of filing: 02.07.2015
(51) Int. Cl.: A61K 31/198, A61K 31/4415, A61K 31/375, A61K 31/194, A61K 31/197, A61K 31/4188, A61K 31/519, A61K 31/675, A61P 3/06, A61K 9/00, A23L 33/00

(54) **BLOOD-FAT REDUCING COMPOSITION AND APPLICATION THEREOF**
BLUTFETTSENKENDE ZUSAMMENSETZUNG UND ANWENDUNG DAVON
COMPOSITION PERMETTANT DE RÉDUIRE LA LIPIDÉMIE ET SON APPLICATION

(30) Priority: 03.07.2014 CN 201410315242
(43) Date of publication of application: 10.05.2017
(73) Proprietor: Wan, Honggui, Jiangsu 210009 (CN); Yue, Maoxing, Chaoyang, Beijing 100101 (CN)
(72) Inventor: Wan, Honggui, Jiangsu 210009 (CN); Yue, Maoxing, Chaoyang, Beijing 100101 (CN)
(74) Representative: Engelhard, Markus
(86) International application number: PCT/CN2015/083178
(87) International publication number: WO 2016/000637

(56) References cited:
- EP-A1- 1 426 043
- WO-A1-2014/139469
- CN-A- 101 953 825
- CN-A- 102 772 407
- CN-A- 104 055 773
- US-A1- 2008 286 254
- CAO, NINGFEN ET AL.: 'Clinical observation of aspartate-ornithine in treatment of hepatitis heparadiposum.' NINGXIA MEDICAL JOURNAL vol. 30, no. 12, 31 December 2008, pages 1108 - 1110, XP008185128
- DATABASE CAPLUS [Online] 12 May 1984 XP055382121 Retrieved from STN Database accession no. 1974:503562 & FROLOVA, I. A.: 'Hypolipemia-inducing action of pyriodoxine.' VOPROSY PITANIYA. 31 December 1974, pages 66 - 8
- DATABASE CAPLUS [Online] 02 April 1997 XP055382125 Retrieved from STN Database accession no. 1997:211514 & EL SWEIDY, M. M. ET AL.: 'The possible alteration in some lipid metabolic aspects in rats during vitamin C and B6 monotherapy.' EGYPTIAN JOURNAL OF PHARMACEUTICAL SCIENCES. vol. 37, no. 1-6, 31 December 1996, pages 405 - 414

## Description

### BACKGROUND

### Technical Field

The present invention relates to the technical fields of health-care products, foods and medicine, in particular to a hypolipidemic composition and use thereof.

### Related Art

Hyperlipidemia means that one or more of lipids in plasma are higher than normal due to lipometabolism or functioning abnormality. It is a systemic disease with the blood total cholesterol (TC) and (or) triglyceride (or) being too high or high-density lipoprotein cholesterol (HDL-C) being too low, which is called dyslipidemia in modern medicine, and often called hyperlipoproteinemia. Most of hyperlipidemia has no any symptom in an initial stage. If not promptly corrected, the hyperlipidemia will generate progressive systemic damage to the body. It will accelerate systemic atherosclerosis, and then cause kidney failure. The current data research shows that the hyperlipidemia can induce the following diseases: coronary heart disease, myocardial infarction, sudden cardiac death, cerebral stroke, hypertension, diabetes, fatty liver, cirrhosis, cholelithiasis, pancreatitis, hyperuricemia and the like.

Amino acids are the most basic substances that constitute the organism protein and are related to life activities, are the basic unit constituting protein molecules in an organism, and are closely related to the life activities of the organism. The amino acids have a special physiological function in antibodies, and are one of indispensable nutrients in the organism. The amino acids can play the following roles through metabolism in the human body: ①synthesizing tissue proteins; ② changing into acids, hormones, antibodies, ceatine and other ammonia-containing substances; ③converting into carbohydrates and fats; ④ oxidating into carbon dioxide, water and urea, and generating energy. Ornithine, which is a non-protein amino acid, can activate key enzymes of urea synthesis - ornithine carbamyl transferase and carbamyl phosphate synthetase, a reaction substrate ornithine can be provided, the synthesis of urea is accelerated through liver ornithine cycle, and the ammonia metabolism is promoted, so that the detoxification of blood ammonia can be realized. Aspartic acid can be directly involved in the urea cycle and involved in the triphosphate cycle and the synthesis of nucleic acids in liver cells firstly, and is conducive to repairing damaged liver cells. In addition, the aspartic acid has an indirect promotion effect on the process of metabolism of tricarboxylic acid in liver cells, and provides intermediate products of energy metabolism to promote the energy generation of liver cells, so that various functions of the damaged liver cells can be rapidly restored.

B vitamins are water-soluble vitamins, including vitamin B1, vitamin B2, vitamin B6, vitamin B12, niacin, pantothenic acid, folic acid and the like, and are indispensable substances that promote in vivo metabolism, and convert sugar, fat, protein and the like into heat. They have a synergistic effect, regulate metabolism, enhance the immune system and promote cell growth and division. B6 may be the most important one of all B vitamins. The body's muscles contain 70% to 80% of B6 in the whole body. B6 plays a key role in protein, lipid and carbohydrate metabolism. Therefore, the loss of a large number of B6 will cause amino acid metabolic disorders. Vitamin B6 is an important coenzyme for amino acid metabolism and synthesis, participates in the metabolism and other physiological processes of unsaturated fatty acids, is a coenzyme of many important enzyme systems in the organism, and is a nutrient necessary for animal normal development and bacteria and yeast reproduction. In addition, vitamin B6 is also a natural diuretic, diuresis plays a detoxification role, and 380 ml or so of urine can be produced by intravenously infusing 5g of vitamin B6. The vitamin B6 is a coenzyme of the human body's amino acid metabolism, a neurotransmitterγ-aminobutyric acid (GABA) and glutamate (Glu). It is known that the vitamin B6 needs to be involved in more than 60 kinds of enzymes in the liver, and plays a very important role in promoting the normal enzyme metabolism of the organism, and the vitamin B6 has a short half life in vivo and is rapidly excreted.

As we all know, the liver plays an important role in lipid digestion, absorption, decomposition, synthesis and transportation and other metabolic processes Lipid modification, synthesis, decomposition, ketone body formation, lipoprotein metabolism, etc. are carried out in the liver. Liver can secrete bile, and bile salts in bile can promote the digestion and absorption of lipids. Triglycerides absorbed from the digestive tract are not assimilated in the liver cells, and are transported to the adipose tissue to be stored; when a person feels hungry, the stored fat is mobilized to the liver and other tissues for catabolism. In the liver, β-oxidation of fatty acids is particularly active, so that a large number of acetyl-CoA(HMG-CoA) is generated, and the HMG-CoA is rapidly decomposed into the ketone body. And lipid transport and transformation are closely related to the liver function. For example, the very low density lipoprotein (VLDL) in plasma is mainly synthesized in the liver, is converted into low-density lipoprotein (LDL) in the plasma, and releases part of high-density lipoprotein (HDL), while an important enzyme - lecithin cholesterol transacylase (LCAT) catalyzing these changes is also synthesized in the liver cells. If the above metabolic reactions are hampered, the lipid content in the liver will change; when the fat is excessively accumulated in the liver, the fatty liver will be caused. Chronic fatty liver can cause liver cell fibrosis, resulting in liver cirrhosis, and damaging the normal function of liver cells. The main occurrence principle of fatty liver is the excessive fat and fatty acids input into the liver, and the anabolism disorders of plasma lipoprotein in the liver.

In the modern society, with the improvement of living standards and the changes in dietary structure of people, the prevalence of hyperlipidemia is on the rise around the world. So far there are many drugs for treatment of hyperlipidemia, and most of them are chemically synthesized chemicals or Chinese herbal compounds formed by some traditional Chinese medicines. Chemical synthetic drugs have a small amount of harmful substances because of its production process, and have toxicity after long-term use, and the late effect is not very satisfactory; the Chinese herbal compound preparation needs to be used for a long time because of its moderate efficacy. Therefore, it is very necessary to develop novel effective hypolipidemic drugs or health-care products to reduce the health hazard of the blood lipid concentration to the human body.

US 2008/0286254 A1 describes compositions containing a licorice polyphenol and other active ingredients, such as antioxidant ingredients, components relating to the absorption or metabolism of lipids, such as lipid absorption inhibitory components or components modifying lipid metabolism.

EP 1 426 043 A1 discloses compositions comprising hydrocolloids and effervescent agents. The document describes compositions comprising, inter alia, aspartic acid, ornithine, pantothenic acid, pyridoxine, vitamin C and folic acid.

Cao, Ningxia Medical Journal, vol. 30, no. 12, 2008, pp. 1108-1110, discloses the use of aspartate and ornithine in the treatmeant of hepatitis hepar adiposum.

Frolova, Voprosy Pitaniya 1974, pp. 66-68 discloses the alteration of levels of cholesterol, total lipids, glycerides and β-lipoproteins by the administration of pyridoxine.

El Sweidy et al., Egyptian Journal of Pharmaceutical Sciences, 1996, vol. 37, no. 1-6, pp. 405-414 discloses that a long-term administration of vitamin C and vitamin B6 induces a significant reduction in serum total cholesterol, triglycerides and non-esterified fatty acid levels.

### SUMMARY

The object of the present invention is to provide a hypolipidemic composition for the above-mentioned technical problems. The hypolipidemic composition is a composition of compound amino acids and vitamins designed for patients suffering from hyperlipidemia, and can effectively reduce blood lipid concentration and awaken the ability of lowering blood lipid of patient bodies.

Another object of the present invention is to provide use of the above-mentioned hypolipidemic composition.

The objects of the present invention are achieved by the following technical solutions:
the hypolipidemic composition comprises ornithine, aspartic acid, vitamin C(VC) and vitamin B6(VB6) and one or more of the following auxiliary components: citric acid, pantothenic acid, biotin and folic acid, in accordance with the appended claims.

The composition comprises the following components by a weight ratio: ornithine: aspartic acid: VB6 = 1: 0.1-1.2: 0.2-1.5, preferably ornithine: aspartic acid: VB6 = 1: 0.3-0.7: 0.4-0.8, and further preferably ornithine: aspartic acid: VB6 = 1: 0.5: 0.6.

The composition also comprises one or more of the following auxiliary components: citric acid, pantothenic acid, biotin and folic acid,
wherein the amounts of the components are as follows by a weight ratio:
ornithine: VC = 1: 0.05-0.2, preferably, ornithine: VC = 1: 0.2-0.8, and further preferably, ornithine: VC = 1: 0.5;
ornithine: citric acid = 1: 0.001 to 0.05, preferably, ornithine: citric acid = 1: 0.001 to 0.03, and further preferably, ornithine: citric acid = 1 : 0.01;
ornithine: pantothenic acid = 1: 0.00001-0.001, preferably, ornithine: pantothenic acid = 1: 0.00005-0.0008, and further preferably, ornithine: pantothenic acid = 1: 0.0005;
ornithine: biotin = 1: 0.000001-0.0005, preferably, ornithine: biotin = 1: 0.000001-0.0001, and further preferably ornithine: biotin = 1: 0.000003;
ornithine: folic acid = 1: 0.000001-0.0005, preferably, ornithine: folic acid = 1: 0.000003-0.0001, and further preferably, ornithine: folic acid = 1: 0.00005.

In the composition, the ornithine refers to L-ornithine, D-omithine or DL-ornithine; and the aspartic acid refers to L-aspartic acid, D-aspartic acid or DL-aspartic acid.

The dosage form of the composition as a health-care product or a drug is any one of pharmaceutically acceptable dosage forms. The preferable dosage forms are granules, tablets, capsules, powders, pills, powders, medicinal liquors, ointments, pellets or granules, membranes, oral solutions and injections.

In a further aspect, the present invention related to the composition according to the present invention for use as a hypolipidemic health-care product or drug in a method of treating hyperlipidemia, in accordance with the appended claims.

Also disclosed is the use of the composition as a food or food alternative. The food additives can be added and applied to various kinds of foods, for example, the food additives can be used as additives for baking pastries, drinks, butter dairy products and the like.

The technical solutions of the invention comprehensively utilize the effect characteristics of the ornithine, the aspartic acid, the vitamin B6 and other substances in accordance with the appended claims; when the ornithine, the aspartic acid and the vitamin B6 are used in combination, the ornithine used as a reaction substrate of the urea cycle rapidly activates the urea cycle in the liver cells, so that the enzyme metabolism in liver is gradually restored, and thus the vitality of the liver can be restored to play a liver protecting role. Aspartic acid can be directly involved in the urea cycle and involved in the triphosphate cycle and the synthesis of nucleic acids in liver cells firstly, and is conducive to repairing damaged liver cells. In addition, the aspartic acid has an indirect promotion effect on the process of cycle metabolism of tricarboxylic acid in liver cells, and provides intermediate products of energy metabolism to promote the energy generation of liver cells, so that various functions of the damaged liver cells can be rapidly restored. Vitamin B6 is an important coenzyme for amino acid metabolism and synthesis, participates in the metabolism and other physiological processes of unsaturated fatty acids, is a coenzyme of many important enzyme systems in the body, and is a nutrient necessary for animal normal development and bacteria and yeast reproduction. And the vitamin B6 is also a natural diuretic, and diuresis plays a detoxification role. Therefore, the ornithine, the aspartate and the vitamin B6 used in combination can effectively repair the damaged liver cells, restore the liver function, and wake up the body's ability to reduce the blood lipid. The three are used in combination to produce phosphatidylcholine to help the emulsification of fat; the liver can also promote the synthesis of bile salts, and digest a large number of triglycerides. The liver is not only an important organ for synthesizing cholesterol in the body, but also cholesterol transported into the liver by the high-density lipoprotein can also be converted in the liver cells and excreted. Part of cholesterol directly used as a bile component together with a bile salt enters the intestine through a bile duct, and the metabolic waste can be smoothly excreted under the action of the vitamin B6 at this time.

The composition provided by the invention has the advantages that:
the curative effect is significant on hyperlipidemia; compared with the existing chemical synthetic drug and traditional Chinese herb compound hypolipidemic method, the composition prepared by the invention not only has less side effect, less recurrence of the disease, and short treatment cycle, but also provides nutritional support for the patients, effectively restores the hypolipidemic function of the body, and promotes enzyme metabolism to achieve the purpose of treating both symptoms and root causes.

The components adopted by the composition provided by the invention can be all derived from food, have high safety, and have no toxic and side effect after being eaten for a long time, but the content ratio of the components is inconsistent with that in food. It is found through experiments that the composition can play a hypolipidemic role while the general food has no such effect.

### DETAILED DESCRIPTION

Hereinafter, the present invention will be further described in detail in conjunction with the examples, but the present invention is not limited to the following examples.

### Example 1 (Comparative Example, not forming part of the invention): Preparation of hypolipidemic sandwich biscuit by using hypolipidemic composition as food additive

The preparation process comprises the following steps:
1. blending wheat flour, eggs, a sweetener, baking soda, vegetable oil and water into dough according to a weight ratio, putting in a refrigerator for cold storage, resting for 1 hour, then taking out, rolling into 2 to 3mm slices, pressing into a shape using a biscuit mold, one being hollow and the other being solid, and preparing into a biscuit ectoderm;
2. putting in an oven for baking, and cooling for standby use;
3. weighing an appropriate amount of jam, the total weight of the jam, L-ornithine, L-aspartic acid and vitamin B6 being 100 parts, evenly mixing the following raw materials in parts by weight: 3 parts of the L-ornithine, 2 parts of the L-aspartic acid, 3 parts of the vitamins B6 and 92 parts of the jam, preparing a food additive, and then smearing the food additive on a hollow part of the biscuit.

### Example 2 (Comparative Example, not forming part of the invention): Preparation of healthcare oral solution

A hypolipidemic health-care oral solution was prepared by the following steps: weighing the following raw materials by weight: 20g of L-ornithine, 5g of L-aspartic acid, 25g of vitamin B6, 15g of vitamin C, 5g of xylitol and 20ml of water, mixing evenly, canning a mixture into a 5-50mL bottle, sealing, and sterilizing at a temperature of 110°C for 20min.

### Example 3 (Comparative Example, not forming part of the invention): Preparation of pharmaceutical composition

A pharmaceutical composition capsule preparation comprises the following components: 70 g of L-ornithine, 60 g of L-aspartic acid, 70 g of vitamin B6, an appropriate amount of 1% polyvinylpyrrolidone ethanol solution and 2 g of magnesium stearate, and 800 capsules were prepared in total.

The preparation method comprises the following steps: weighing the L-ornithine, the L-aspartic acid and the vitamin B6, mixing evenly, preparing a soft material with 40 mL of the 1% polyvinylpyrrolidone ethanol solution, granulating with a 20-mesh sieve, drying until the moisture does not exceed 3%, adding 2g of the magnesium stearate, mixing evenly, granulating with the 20-mesh sieve, sub-packaging, polishing and packaging, thus obtaining a capsule preparation through the above process.

### Example 4 (Comparative Example, not forming part of the invention): Preparation of pharmaceutical composition

A pharmaceutical composition tablet comprises the following components: 70 g of L-ornithine, 60 g of L-aspartic acid, 70 g of vitamin B6, an appropriate amount of 1% polyvinylpyrrolidone ethanol solution and 1g of magnesium stearate, and 1000 tablets were prepared in total.

The preparation method comprises the following steps: weighing the L-ornithine, the L-aspartic acid and the vitamin B6, mixing evenly, preparing a soft material with 20 mL of the 1% polyvinylpyrrolidone ethanol solution, granulating with a 20-mesh sieve, drying, adding 1g of magnesium stearate, mixing evenly, granulating with the 20-mesh sieve, tabletting, polishing and packaging, thus obtaining a composition tablet through the above process.

### Example 5: Preparation of pharmaceutical composition

A pharmaceutical composition comprises 250 mL of a mixed substance injection containing 6 g of L-ornithine, 4 g of L-aspartic acid, 6 g of vitamin B6, 3.5 g of vitamin C, and0.lmg of biotin, and was added into 250mL of a 5% glucose and sodium chloride injection (0.9% sodium chloride injection used for diabetic patients).

### Example 6:

A pharmaceutical composition granule comprises the following components: 70 g of L-ornithine, 60 g of L-aspartic acid, 70 g of vitamin B6, 0.5 g of citric acid, 5 mg of pantothenic acid, 0.2 mg of biotin, 0.3 mg of folic acid and 15g of vitamin C. An appropriate amount of 20% ethanol solution was used as a wetting agent, so that the granule was prepared,

The preparation method comprises the following steps: weighing the above-mentioned components, mixing evenly, adding 40ml of 20% ethanol solution, preparing a soft material, preparing the granule by an extrusion-spheronization method, and drying until the moisture is not more than 3%.

### Example 7:

A pharmaceutical composition granule comprises the following components: 70 g of L-ornithine, 60 g of D-aspartic acid, 70 g of vitamin B6, 0.5 g of citric acid, 0.2 mg of biotin and 20 g of vitamin C. An appropriate amount of 20% ethanol solution was used as a wetting agent, so that the granule was prepared.

The preparation method comprises the following steps: weighing the above-mentioned components, mixing evenly, adding 40ml of 20% ethanol solution, preparinga soft material, preparing the granule by an extrusion-spheronization method, and drying until the moisture is not more than 3%.

### Example 8:

A pharmaceutical composition granule comprises the following components: 70g of DL-ornithine, 50g of L-aspartic acid, 60 g of vitamin B6, 30mg of pantothenic acid, 0.5mg of biotin and 20 g of vitamin C. An appropriate amount of 20% ethanol solution was used as a wetting agent, so that the granule was prepared.

The preparation method comprises the following steps: weighing the above-mentioned components, mixing evenly, adding 40ml of 20% ethanol solution, preparing a soft material, preparing the granule by an extrusion-spheronization method, and drying until the moisture is not more than 3%.

### Example 9:

A pharmaceutical composition granule comprises the following components: 70g of L-ornithine, 50g of DL-aspartic acid, 60 g of vitamin B6, 30mg of pantothenic acid, 3mg of folic acid and 5 g of vitamin C. An appropriate amount of 20% ethanol solution was used as a wetting agent, so that the granule was prepared.

The preparation method comprises the following steps: weighing the above-mentioned components, mixing evenly, adding 40ml of 20% ethanol solution, preparing a soft material, preparing the granule by an extrusion-spheronization method, and drying until the moisture is not more than 3%.

### Example 10 (Comparative Example, not forming part of the invention):

A pharmaceutical composition granule comprises the following components: 70g of L-ornithine, 50g of DL-aspartic acid, 35g of vitamin B6 and 45 g of vitamin C. An appropriate amount of 20% ethanol solution was used as a wetting agent, so that the granule was prepared.

The preparation method comprises the following steps: weighing the above-mentioned components, mixing evenly, adding 40ml of 20% ethanol solution, preparing a soft material, preparing the granule by an extrusion-spheronization method, and drying until the moisture is not more than 3%.

### Example 11: Hypolipidemic animal experiment of composition provided by the invention

1. Experimental materials: 150 healthy SD male and female rats in clean grade, with body weight of (150.2 ± 8.9) g.
2. Feed: general feed, high-fat feed formula: 72.7% of basic feed, 2% of cholesterol, 5% of egg yolk powder, 10% of lard, 0.2% of propylthiouracil, 10% of sucrose and 0.1% of sodium cholate.
3. Grouping and administration of animals: the purchased experimental rats were adaptively fed for 1 week in a lab, original numbers were assigned according to the weighing order, and the rats were randomly divided into a blank control group, a model control group, a pharmaceutical composition (respectively prepared as in Examples 3, 6, 8 and 10, and added with 50ml of water per 10.g to be dissolved to obtain a solution) low-dose group (5ml / kg. d), a middle-dose group (10ml / kg-d), a high-dose group (15ml / kg. d), a positive drug (simvastatin) control group (1.5mg / kg, volume: 10ml / kg) by looking up a random number table, and there were 10 rats in each group. From the day of experiment, the blank control group and the model control group were intragastrically administered with normal saline (volume: 10ml / kg), the administration group was intragastrically administered with corresponding drugs once a day, the body weight was weighed once a week, and the dosage was adjusted according to the body weight. The blank control group was fed with the normal feed, the other groups was fed with high-fat feed. During the experiment, free feeding, free drinking, continuous molding and drug administration for prevention were carried out for 4 weeks.
4. Observation indexes: after the last administration, the rats fasted for 12h without fasting water. The rats were anesthetized with 3% pentobarbital sodium, blood was collected from the abdominal aorta, serum was centrifugally separated, and the serum total cholesterol (TC), triglyceride (TG), high-density lipoprotein cholesterol (HDL-C), low-density lipoprotein cholesterol (LDL-C), alanine aminotransferase (ALT) and aspartate aminotransferase (AST) values were measured.

### 5. Results

### a) Effect of the composition provided by the invention on blood lipid levels in rats

As shown in Table 1, compared with the normal group, the levels of TC, TG and LDL-C in the serum of the rats in the model group were significantly increased (P <0.05), showing typical lipid metabolism disorder, thus indicating that an experimental hyperlipidemia model was successfully replicated. (The compositions of Examples 3 and 10 do not form part of the invention.)
Compared with the model group, the contents of TC, TG and LDL-C in the serum in the treatment group were significantly decreased (P <0.05), and the HDL-C content of the serum was significantly increased (P <0.01) in a dose-dependent manner.

**Table 1 Changes of blood lipids of rats in various groups(X̅±S, n=10)**

| Groups | | TC (mmol/L) | TG (mmol/L) | HDL-C (mmol/L) | LDL-C (mmol/L) |
|---|---|---|---|---|---|
| Normal control group | | 1.63 ± 0.53 | 0.71 ± 0.42 | 1.36 ± 0.25 | 0.26 ± 0.27 |
| Model control group | | 12.34 ± 3.17 | 1.20 ± 0.45 | 0.94 ± 0.36 | 5.67 ± 3.37 |
| Example 3: Drug | Low-dose group Middle-dose group High-dose group | 6.19 ± 1.70 | 0.70 ± 0.27 | 1.56 ± 0.15 | 2.40 ± 1.87 |
| | | 5.91 ± 3.01 | 0.67 ± 0.20 | 1.64 ± 0.10 | 1.88 ± 1.49 |
| | | 1.65 ± 0.21 | 0.57 ± 0.19 | 1.66 ± 0.69 | 0.99 ± 0.83 |
| Example 6: Drug | Low-dose group Middle-dose group High-dose group | 6.17 ± 1.62 | 0.69 ± 0.22 | 1.53 ± 0.11 | 2.33 ± 1.76 |
| | | 4.67 ± 3.18 | 0.60 ± 0.15 | 1.62 ± 0.08 | 1.85 ± 1.40 |
| | | 1.61 ± 0.21 | 0.53 ± 0.16 | 1.65 ± 0.61 | 0.97 ± 0.71 |
| Example 8: Drug | Low-dose group Middle-dose group High-dose group | 6.05 ± 1.71 | 0.60 ± 0.24 | 1.46 ± 0.13 | 2.28 ± 1.80 |
| | | 4.51 ± 3.21 | 0.52 ± 0.19 | 1.55 ± 0.10 | 1.80 ± 1.46 |
| | | 1.52 ± 0.23 | 0.43 ± 0.18 | 1.59 ± 0.61 | 0.95 ± 0.73 |
| Example 10: Drug | Low-dose group Middle-dose group High-dose group | 6.10 ± 1.69 | 0.65 ± 0.27 | 1.49 ± 0.10 | 2.29 ± 1.83 |
| | | 4.58 ± 3.25 | 0.58 ± 0.17 | 1.59 ± 0.15 | 1.83 ± 1.50 |
| | | 1.56 ± 0.19 | 0.50 ± 0.16 | 1.63 ± 0.65 | 0.96 ± 0.76 |
| Positive drug control group | | 8.42 ± 4.79 | 0.49 ± 0.18 | 1.23 ± 0.17 | 1.68 ± 1.91 |

### b) Effect of the composition provided by the invention on liver function of rats

The liver function could be reflected by ALT and AST in the serum. The levels of ALT and AST in the high-fat feed group were significantly higher than those in the basic feed group (P <0.01), indicating that there was a certain damage to the liver. The levels of ALT and AST in the serum in each administration group were lower than those in the model control group, and there was a significant difference between the middle-dose group, the high-dose group and the model control group (P <0.01), indicating that the composition provided by the invention has a significant effect on protection of liver function, and a significant protecting effect on the organism. (The compositions of Example 3 and 10 do not form part of the invention.)

**Table 2 Changes of liver function of rats in each group (X̅±S, n=10)**

| Groups | | Dose (ml/kg-d) | ALT (U/L) | AST(U/L) |
|---|---|---|---|---|
| | Normal control group | - | 40.39 ± 6.80 | 19.74 ± 3.16 |
| | Model control group | - | 55.44 ± 2.88 | 31.51 ± 5.68 |
| Example 3 Drug | Low-dose group | 5 | 48.19 ± 5.52 | 26.71±1.66 |
| | Middle-dose group | 10 | 44.33 ± 3.94 | 20.88 ± 7.01 |
| | High-dose group | 15 | 42.55 ± 4.49 | 18.39 ± 5.70 |
| Example 6 Drug | Low-dose group | 5 | 48.16 ± 5.57 | 26.66±1.64 |
| | Middle-dose group | 10 | 44.27 ± 3.91 | 20.81 ± 7.11 |
| | High-dose group | 15 | 42.46 ± 4.47 | 18.37 ± 5.77 |
| Example 8 Drug | Low-dose group | 5 | 48.05 ± 5.59 | 26.63±1.60 |
| | Middle-dose group | 10 | 44.19 ± 3.95 | 20.77 ± 7.15 |
| | High-dose group | 15 | 42.43 ± 4.40 | 18.34 ± 5.79 |
| Example 10 Drug | Low-dose group | 5 | 48.18 ± 5.57 | 26.67 ± 1.65 |
| | Middle-dose group | 10 | 44.22 ± 3.99 | 20.84 ± 7.11 |
| | High-dose group | 15 | 42.49 ± 4.47 | 18.38 ± 5.70 |
| | Positive drug control group | 1.5mg/Kg-d | 45.85 ± 5.21 | 22.32 ± 7.56 |

The contents of TC, TG and LDL-C were significantly decreased while the content of HDL-C was increased after the hyperlipidemia rats were drenched with the composition provided by the present invention; and the increase in ALT and AST in the serum caused by the high-fat feed was inhibited. It was indicated that the composition provided by the invention promoted fatty acid metabolism, improved intrahepatic lipid metabolism, protected liver cells and regulated lipids and protected liver, and was especially suitable for hyperlipidemia patients with high risk caused by liver function damage. Pharmacological experiments proved that the ornithine, the aspartic acid and the vitamin B6 had a synergistic effect, and could enhance the efficacy each other.

### Example 12 (Comparative Example): Effect of composition provided by the invention in clinical treatment

### 1. Case selection

Patients with more than 5.7mmol / L of serum total cholesterol (TC) and (or) more than 1.7mmol / L of triglyceride (TG) were selected as observation objects, and there were 24 cases in total, including 14 males and 10 females, aged 26 to 69 years.

### 2. Diagnostic criteria

Referring to "Internal Medicine"published by People's Medical Publishing House in 2010, the diagnostic criteria of hyperlipidemia was as follows: serum total cholesterol(TC)was more than or equal to5.72mmol/L, triglyceride (TG)was more than or equal to1.70mmol/L, low-density lipoprotein cholesterol (LDL-C)was more than or equal to3.10mmol/L, and high-density lipoprotein(HDL-C) was less than or equal tol.04mmol/L for males, and less than or equal to 1. 17mmol/L for females.

### 3. Treatment method

All patients were prohibited from drinking alcohol and eating excessive lipid food at dinner on the day before blood drawing, and TC, TG, HDL-C, and LDL-C were measured by drawing venous blood after the patients fasted for more than 12 hours. Then, the composition prepared as in Example 3 was administered three times a day for four continuous weeks, two pills were taken once, each pill contained about 0.25 g of the composition, and spicy food, especially alcohol was restrained during treatment.

### 4. Efficacy evaluation standards

Markedly effective: After treatment, the blood lipid was detected to conform to any one of the following standards: TC was decreased by more than or equal to20%, or TG was decreased by more than or equal to 40%, or HDL-C was increased by more than or equal to0.26mmol/L, or HDL-C/LDL-C was decreased by more than or equal to20%, or LDL-C was decreased by more than or equal to30%.

Effective: the blood lipid was detected to conform to any one of the following standards: TC was decreased by more than or equal to10% and less than 20%, or TG was decreased by more than or equal to20% and less than 40%, or HDL-C was increased by more than or equal to0.14mmol/L and less than 0.26mmol/L, or HDL-C/LDL-C was decreased by more than or equal to10% and less than 20%, or LDL-C was decreased by more than or equal to20%.

Ineffective: the blood lipid was obviously improved or the improvement on the blood lipid did not conform to the standards before and after treatment.

### 5. Treatment effects

After 24 cases of patients were treated with the composition provided by the invention for two weeks, the composition was markedly effective in 8 cases, effective in 11 cases, and ineffective in 5 cases, and TC and TG were decreased to different degrees in the5 ineffective cases. After the composition provided by the invention was continuously administered for two weeks, the composition was markedly effective in 14 cases, effective in 9 cases, and ineffective in 1 case, the inefficiency was 4.2%, and the total efficiency was 95.8%.

### 6. Description of specific cases

(1) Gao, male, 33 years old, hyperlipidemia. Before treatment, the total cholesterol (TC) was 6.31mmol / L and the triglyceride (TG) was 2.16mmol / L. The total cholesterol (TC) was decreased to 4.92 mmol / L and the triglyceride (TG) was decreased to 0.99 mmol / L two weeks after administration of the composition.
(2) Xu, female, 69 years old, hyperlipidemia. Before treatment, the total cholesterol (TC) was 5.79mmol / L and the triglyceride (TG) was 4.12mmol / L. The total cholesterol (TC) was decreased to 5.46mmol / L and the triglyceride (TG) was decreased to 1.50mmol / L two weeks after administration of the composition.

## Claims

1. A hypolipidemic composition, **characterized by** comprising ornithine, aspartic acid,vitamin B6, vitamin C, and one or more of the following auxiliary components:
citric acid, pantothenic acid, biotin and folic acid;
by a weight ratio:
ornithine: aspartic acid: VB6 = 1: 0.1-1.2: 0.2-1.5;
ornithine: VC = 1: 0.05-1.2;
ornithine: citric acid = 1: 0.001-0.05;
ornithine: pantothenic acid = 1: 0.00001-0.001;
ornithine: biotin = 1: 0.000001-0.0005;
ornithine: folic acid = 1: 0.000001-0.0005.

2. The composition according to claim 1, **characterized by** comprising the following components by a weight ratio ornithine: aspartic acid: VB6 = 1: 0.3-0.7: 0.4-0.8.

3. The composition according to claim 2, **characterized by** comprising the following components by a weight ratio ornithine: aspartic acid: VB6 = 1: 0.5: 0.6.

4. The composition according to claim 1, **characterized by** comprising the following components by a weight ratio ornithine: VC = 1: 0.2-0.8.

5. The composition according to claim 4, **characterized by** comprising the following components by a weight ratio ornithine: VC = 1: 0.5.

6. The composition according to claim 1, **characterized by** comprising the following components by a weight ratio ornithine: citric acid = 1: 0.001-0.03.

7. The composition according to claim 6, **characterized by** comprising the following components by a weight ratio ornithine: citric acid = 1 : 0.01.

8. The composition according to claim 1, **characterized by** comprising the following components by a weight ratio ornithine: pantothenic acid = 1: 0.00005-0.0008.

9. The composition according to claim 8, **characterized by** comprising the following components by a weight ratio ornithine: pantothenic acid = 1: 0.0005.

10. The composition according to claim 1, **characterized by** comprising the following components by a weight ratio ornithine: biotin = 1: 0.000001-0.0001, preferably by a weight ratio ornithine: biotin = 1: 0.000003.

11. The composition according to claim 1, **characterized by** comprising the following components by a weight ratio ornithine: folic acid = 1: 0.000003-0.0001, preferably by a weight ratio ornithine: folic acid = 1: 0.00005.

12. The composition according to any one of claims 1 to 11, **characterized in that** the ornithine refers to L-ornithine, D-ornithine or DL-ornithine; and the aspartic acid refers to L-aspartic acid, D-aspartic acid or DL-aspartic acid.

13. The composition according to any of claims 1 to 11, **characterized in that** the dosage form of the composition as a health-care product or a drug is any one of pharmaceutically acceptable dosage forms

14. The composition according to claim 13, **characterized in that** the dosage forms are granules, tablets, capsules, pulvis, pills, powders, medicinal liquors, ointments, pellets, granules, membranes, oral solutions and injections.

15. The composition according to any of claims 1 to 11 for use as a hypolipidemic health-care product or drug in a method of treating hyperlipidemia.

## Patentansprüche

1. Hypolipidämische Zusammensetzung, **dadurch gekennzeichnet, dass** sie Ornithin, Asparaginsäure, Vitamin B6, Vitamin C und einen oder mehrere der folgenden zusätzlichen Bestandteile umfasst:
Zitronensäure, Pantothensäure, Biotin und Folsäure;
in einem Gewichtsverhältnis:
Ornithin: Asparaginsäure: VB6 = 1: 0,1-1,2: 0,2-1,5;
Ornithin: VC = 1: 0,05-1,2;
Ornithin: Zitronensäure = 1: 0,001-0,05;
Ornithin: Pantothensäure = 1: 0,00001-0,001;
Ornithin: Biotin = 1: 0,000001-0,0005;
Ornithin: Folsäure = 1: 0,000001-0,0005.

2. Zusammensetzung nach Anspruch 1, **dadurch gekennzeichnet, dass** sie die folgenden Bestandteile in einem Gewichtsverhältnis Ornithin: Asparaginsäure: VB6 = 1: 0,3-0,7: 0,4-0,8 umfasst.

3. Zusammensetzung nach Anspruch 2, **dadurch gekennzeichnet, dass** sie die folgenden Bestandteile in einem Gewichtsverhältnis Ornithin: Asparaginsäure: VB6 = 1: 0,5: 0,6 umfasst.

4. Zusammensetzung nach Anspruch 1, **dadurch gekennzeichnet, dass** sie die folgenden Bestandteile in einem Gewichtsverhältnis Ornithin: VC = 1: 0,2-0,8 umfasst.

5. Zusammensetzung nach Anspruch 4, **dadurch gekennzeichnet, dass** sie die folgenden Bestandteile in einem Gewichtsverhältnis Ornithin: VC = 1: 0,5 umfasst.

6. Zusammensetzung nach Anspruch 1, **dadurch gekennzeichnet, dass** sie die folgenden Bestandteile in einem Gewichtsverhältnis Ornithin: Zitronensäure = 1: 0,001-0,03 umfasst.

7. Zusammensetzung nach Anspruch 6, **dadurch gekennzeichnet, dass** sie die folgenden Bestandteile in einem Gewichtsverhältnis Ornithin: Zitronensäure = 1 : 0,01 umfasst.

8. Zusammensetzung nach Anspruch 1, **dadurch gekennzeichnet, dass** sie die folgenden Bestandteile in einem Gewichtsverhältnis Ornithin: Pantothensäure = 1: 0,00005-0,0008 umfasst.

9. Zusammensetzung nach Anspruch 8, **dadurch gekennzeichnet, dass** sie die folgenden Bestandteile in einem Gewichtsverhältnis Ornithin: Pantothensäure = 1: 0,0005 umfasst.

10. Zusammensetzung nach Anspruch 1, **dadurch gekennzeichnet, dass** sie die folgenden Bestandteile in einem Gewichtsverhältnis Ornithin: Biotin = 1: 0,000001-0,0001, vorzugsweise in einem Gewichtsverhältnis Ornithin: Biotin = 1: 0,000003, umfasst.

11. Zusammensetzung nach Anspruch 1, **dadurch gekennzeichnet, dass** sie die folgenden Bestandteile in einem Gewichtsverhältnis Ornithin: Folsäure = 1: 0,000003-0,0001, vorzugsweise in einem Gewichtsverhältnis Ornithin: Folsäure = 1: 0,00005, umfasst.

12. Zusammensetzung nach einem der Ansprüche 1 bis 11, **dadurch gekennzeichnet, dass** sich das Ornithin auf L-Ornithin, D-Ornithin oder DL-Ornithin bezieht; und die Asparaginsäure sich auf L-Asparaginsäure, D-Asparaginsäure oder DL-Asparaginsäure bezieht.

13. Zusammensetzung nach einem der Ansprüche 1 bis 11, **dadurch gekennzeichnet, dass** die Dosierungsform der Zusammensetzung als ein Gesundheitsprodukt oder als ein Arzneimittel eine von pharmazeutisch akzeptablen Dosierungsformen ist.

14. Zusammensetzung nach Anspruch 13, **dadurch gekennzeichnet, dass** die Dosierungsformen Granulate, Tabletten, Kapseln, Pulvis, Pillen, Pulver, medizinische Flüssigkeiten, Salben, Pellets, Granula, Membranen, orale Lösungen und Injektionen sind.

15. Zusammensetzung nach einem der Ansprüche 1 bis 11 zur Verwendung als ein hypolipidämisches Gesundheitsprodukt oder Arzneimittel bei einem Verfahren zum Behandeln von Hyperlipidämie.

## Revendications

1. Composition hypolipidémiante, **caractérisée en ce qu'**elle comprend de l'ornithine, de l'acide aspartique, de la vitamine B6, de la vitamine C, et un ou plusieurs des composants auxiliaires suivants :
acide citrique, acide pantothénique, biotine et acide folique ;
avec un rapport en poids :
ornithine : acide aspartique : VB6 = 1 : 0,1-1,2 : 0,2-1,5 ;
ornithine : VC = 1 : 0,05-1,2 ;
ornithine : acide citrique = 1 : 0,001-0,05 ;
ornithine : acide pantothénique = 1 : 0,00001-0,001 ;
ornithine : biotine = 1 : 0,000001-0,0005 ;
ornithine : acide folique = 1 : 0,000001-0,0005.

2. Composition selon la revendication 1, **caractérisée en ce qu'**elle comprend les composants suivants avec un rapport en poids ornithine : acide aspartique : VB6 = 1 : 0,3-0,7 : 0,4-0,8.

3. Composition selon la revendication 2, **caractérisée en ce qu'**elle comprend les composants suivants avec un rapport en poids ornithine : acide aspartique : VB6 = 1 : 0,5 : 0,6.

4. Composition selon la revendication 1, **caractérisée en ce qu'**elle comprend les composants suivants avec un rapport en poids ornithine : VC = 1 : 0,2-0,8.

5. Composition selon la revendication 4, **caractérisée en ce qu'**elle comprend les composants suivants avec un rapport en poids ornithine : VC = 1 : 0,5.

6. Composition selon la revendication 1, **caractérisée en ce qu'**elle comprend les composants suivants avec un rapport en poids ornithine : acide citrique = 1 : 0,001-0,03.

7. Composition selon la revendication 6, **caractérisée en ce qu'**elle comprend les composants suivants avec un rapport en poids ornithine : acide citrique = 1 : 0,01.

8. Composition selon la revendication 1, **caractérisée en ce qu'**elle comprend les composants suivants avec un rapport en poids ornithine : acide pantothénique = 1 : 0,00005-0,0008.

9. Composition selon la revendication 8, **caractérisée en ce qu'**elle comprend les composants suivants avec un rapport en poids ornithine : acide pantothénique= 1 : 0,0005.

10. Composition selon la revendication 1, **caractérisée en ce qu'**elle comprend les composants suivants avec un rapport en poids ornithine : biotine = 1 : 0,000001-0,0001, de préférence avec un rapport en poids ornithine : biotine = 1 : 0,000003.

11. Composition selon la revendication 1, **caractérisée en ce qu'**elle comprend les composants suivants avec un rapport en poids ornithine : acide folique = 1 : 0,000003-0,0001,
de préférence avec un rapport en poids ornithine : acide folique = 1 : 0,00005.

12. Composition selon l'une quelconque des revendications 1 à 11, **caractérisée en ce que** l'ornithine désigne la L-ornithine, la D-ornithine ou la DL-ornithine ; et l'acide aspartique désigne l'acide L-aspartique, l'acide D-aspartique ou l'acide DL-aspartique.

13. Composition selon l'une quelconque des revendications 1 à 11, **caractérisée en ce que** la forme pharmaceutique de la composition en tant que produit de soin ou médicament est l'une quelconque parmi des formes pharmaceutiques pharmaceutiquement acceptables.

14. Composition selon la revendication 13, **caractérisée en ce que** les formes pharmaceutiques sont des granules, des comprimés, des capsules, une poudre fine, des pilules, des poudres, des liqueurs médicinales, des pommades, des pastilles, des granules, des membranes, des solutions orales et des injections.

15. Composition selon l'une quelconque des revendications 1 à 11 pour utilisation en tant que produit de soin ou médicament hypolipidémiant dans un procédé de traitement de l'hyperlipidémie.
